# EUROPEAN PATENT SPECIFICATION

(11) **EP 1 996 067 B1**
(45) Date of publication and mention of the grant of the patent: **10.04.2013**
(21) Application number: 06851371.2
(22) Date of filing: 17.05.2006
(51) Int. Cl.: G01N 33/94, G01N 1/40, A61B 5/00

(54) **MONITORING CYCLOSPORINE IN SALIVA**
ÜBERWACHUNG VON CYCLOSPORIN IN SPEICHEL
SURVEILLANCE DE LA CYCLOSPORINE DANS LA SALIVE

(30) Priority: 19.05.2005 US 682442 P
(43) Date of publication of application: 03.12.2008
(73) Proprietor: The Board Of Governors For Higher Education, Providence, RI 02908 (US)
(72) Inventor: AKHLAGHI, Fatemeh, Wakefield, RI 02875 (US); MENDONZA, Anisha, E., Plainsboro New Jersey 08536 (US)
(74) Representative: Harrison Goddard Foote
(86) International application number: PCT/US2006/018961
(87) International publication number: WO 2008/121096

(56) References cited:
- US-A- 5 354 654
- US-A- 5 698 448
- US-B1- 6 190 873
- ANISHA MENDOZA ET AL: "Determination of Cyclosporine in Saliva using Liquid Chromatography-Tandem Mass Spectrometry Therapeutic Drug Monitoring", THERAPEUTIC DRUG MONITORING, LIPPINCOTT WILLIAMS AND WILKINS, NEW YORK, NY, US, vol. 26, no. 5, 1 October 2004 (2004-10-01), pages 569-575, XP008128709, ISSN: 0163-4356
- MEULENBERG E P ET AL: "The effect of pretreatment of saliva on steroid hormone concentrations.", JOURNAL OF CLINICAL CHEMISTRY AND CLINICAL BIOCHEMISTRY. ZEITSCHRIFT FÜR KLINISCHE CHEMIE UND KLINISCHE BIOCHEMIE DEC 1990 LNKD- PUBMED:2081963, vol. 28, no. 12, December 1990 (1990-12), pages 923-928, XP002651801, ISSN: 0340-076X
- MENDONZA ET AL.: 'Determination of Cyclosporine in Saliva using Liquid Chromatography-Tandem Mass Spectrometry Therapeutic Drug Monitoring' vol. 26, no. 5, October 2004, pages 569 - 575, XP008128709

## Description

### BACKGROUND OF THE INVENTION

Cyclosporine is an immunosuppressive agent commonly used for the prevention of rejection of organs after organ transplants and for the treatment of autoimmune diseases including psoriasis, rheumatoid arthritis etc. Because of significant toxicity, the blood concentration of cyclosporine must be monitored by measuring blood concentration on a routine basis in patients receiving the drug.

Saliva is an oral fluid that has been described as an ultra-filtrate of plasma. Saliva has recently been well established as a diagnostic tool in detecting many of the molecules that are found in plasma and at levels comparable to those found in blood. Therefore, by testing saliva, one can obtain similar information on the concentration of drugs without the need to collect a specimen invasively.

Many commercial methods are now available for the salivary measurement of ethanol, drugs of abuse, cortisol and steroid hormones however as far as the published literature goes, there has not been any commercialization for assay methods for the measurement of pharmacological agents in saliva.

Therapeutic drug monitoring in saliva offers many advantages over a blood- or plasma-based method. No venipuncture is required as is the case with blood collection, and it can be performed, with minimal training, by the patient or a caregiver. The finger prick method of blood collection seems to be a better alternative to venipuncture; however, it still remains invasive, and it also may be difficult to obtain sufficient quantity of blood from some patients (i.e. patients with collapsible veins or small children). Saliva monitoring requires small amounts of sample (less than 1 mL) and is ideal for drug monitoring in children and patients with difficult venous access. Drugs enter saliva predominately *via* passive diffusion, a process that is limited to lipophilic and unionized drugs. The diffusion of drugs between blood and saliva is also limited to the unbound fraction of the drug because the "protein-bound drug complex" is unable to pass through small channels in the capillaries of salivary glands. It is therefore conceivable to believe that the salivary concentration will reflect the unbound and pharmacologically active species of a drug.

The unbound concentration of CsA is believed to be responsible for its immunosuppressive action. However, it is practically impossible to measure the CsA unbound concentration in plasma or blood because of difficulties associated with the measurement of unbound concentration of such lipophilic molecules. It has been speculated that saliva monitoring is ideal for measuring neutral lipophilic compounds such as CsA. The availability of a saliva-based method is therefore essential to measure the salivary concentration of CsA and to explore its correlation with the total or unbound concentration.

The method described in the first assay for the quantitative of cyclosporine in saliva used a sensitive, specific and non-radioactive technique. There was another method published in 1988 (Coates JE, Lam SF, McGaw T, Radioimmunoassay of Salivary Cyclosporine with Use of ¹²⁵Iodine-Labelled Cyclosporine, Clin Chem 434/8, 1545-1551) that has described an assay for the measurement of cyclosporine in saliva by modification of a polyclonal

radioimmunoassay (RIA) kit originally developed for the measurement of cyclosporine in blood. This original blood measurement kit is no longer available commercially and therefore it is not possible to duplicate the assay described in the publication mentioned above. The blood kit included an assay for measuring CsA in saliva was a polyclonal [¹²⁵I] cyclosporine radioimmunoassay (RIA) method. The major disadvantage of this method was nonspecificity because of cross-reactivity with CsA metabolites and the requirement to produce [¹²⁵I] CsA to replace the original [³H] CsA.

Mass spectrometry (MS) coupled with HPLC (LC-MS/MS) is increasingly used for the measurement of CsA in whole blood. The LC-MS/MS methodology offers many advantages to an immunoassay based method including superior sensitivity, specificity and a rapid turnaround time. The LC-MS/MS technique was therefore used in the development of an assay for CsA measurement in saliva.

Mendonza A et al (2004), Ther Drug Monit, Vol 26 No. 5: 569-575, report a method for determination of cyclosporine in saliva using liquid chromatography-tandem mass spectrometry.

Meulenberg, E. P. M. M. and J.A. Hofman (1990), J Clin. Chem. Clin. Biochem. Vol 28: 923-928, report a study of the effect of pretreatment of saliva on steroid hormone concentrations.

### SUMMARY OF THE INVENTION

A method for measuring the concentration of the immunosuppressive agent, cyclosporine, in human saliva samples.

The monitoring cyclosporine is saliva can be a substitute for the monitoring cyclosporine in blood. The method is sensitive with a lower limit of quantification of 1 ng/mL allowing the measurement of very low concentrations observed in saliva. The analytical method is specific for the parent cyclosporine molecule so the measured concentrations represent the concentration of parent drug not inactive metabolites.

The devised extraction method for cyclosporine from saliva matrix is novel because of the followings of the order of addition of saliva to extraction solvents. It is important that the saliva be added to the solvent. Additionally, the pre-treatment of saliva with sonication significantly greatly improved the recovery and consistency of cyclosporine extraction from saliva

It is an object of this invention to provide an analytical testing method to determine the amount of cyclosporine in saliva.

These and other features and objectives of the present invention will now be described in greater detail with reference to the accompanying drawings, wherein:

### BRIEF DESCRIPTION OF THE DRAWINGS

Figs. 1A and 1B are specificity of the assay in blank saliva and blank saliva spiked with internal standard, respectively;
Fig. 2 is a chromatogram of cyclosporine D:
Fig 3 is a plot of cyclosporine concentrations in whole blood versus saliva;
Fig. 4A and 4B are plots of association between blood and saliva concentrations; and
Fig 5 is a plot of concentrations of cyclosporine in blood and saliva.

### DETAILED DESCRIPTION OF THE INVENTION

Described herein is a method for measuring the amount of the immunosuppresive agent, cyclosporine in human saliva samples. The validation procedure of the assay to measure cyclosporine in saliva according to the guidelines set by the Food and Drug Administration of the United States is also described herein. A correlation was found of the results between saliva concentrations with blood and plasma concentrations in samples obtained from kidney transplant recipients.

Saliva measurement of CsA presented a number of challenging issues that had to be overcome for successful method development. First, the concentration of CsA in saliva would be much lower than the CsA concentration in whole blood. In a study of 36 kidney transplant recipients, the mean ± SD of trough concentration of CsA in saliva was 8.3 ± 5.2 µg/L using the RIA method described earlier. This concentration is much lower than the expected through or minimum concentrations in whole blood. Using the ammonium adducts for CsA and CsC, a Lower Limit of Quantification (LLOQ) of 1 µg/L, which is more suitable for measurement of CsA in saliva, was obtained.

The current assay is specific because it measures only the concentration of parent cyclosporine as opposed to the polyclonal RIA that measures the concentration of parent and metabolites.

Cyclosporine (CsA) is a potent and effective immunosuppressive agent widely used following organ transplantation or for the treatment of autoimmune diseases. Upon complex formation with cyclophilin, CsA inhibits the activation of calcineurin, a calcium/calinodulin-related phosphatase, thus preventing the translocation of a transcription factor; nuclear factor of activated T cells (NF-AT). Cyclosporine also inhibits activation of another transcription factor, NF-KB, resulting in suppression of T-cell activation by the means of inhibiting in terleukin-2 gene expression.

Because of the narrow therapeutic index and significant intra- and interindividual variability in the pharmacokinetic characteristics of CsA, the therapeutic concentrations of this drug must be routinely monitored. However, despite routine monitoring, many patients experience organ rejection and/or adverse effects including nephrotoxicity, hepatotoxicity, neurotoxicity, and gingival hyperplasia.

Cyclosporine is more than 99% bound to blood cells and plasma proteins, leaving a very small fraction of unbound (free) drug. It is generally believed that the pharmacological action of a highly protein-bound drug is dependent on the drug concentration at the receptor site, which is directly related to the concentration of the unbound drug in plasma. In addition, it has previously been shown that the unbound CsA concentration in plasma was associated to a greater extent than the total concentration with the incidence and severity of heart transplant rejection. Determining CsA unbound concentration may therefore prove beneficial in the management of CsA therapy.

Several methods including ultracentrifugation, erythrocyte partitioning, equilibrium dialysis using stainless steel chambers, and microdialysis have been used to separate and measure the unbound concentration of CsA in plasma. However, because of the hydrophobic nature of the CsA molecule, many of these methods, with the exception of equilibrium dialysis, provide unreliable estimates for the CsA unbound concentration. Furthermore, the equilibrium dialysis technique using radiolabeled CsA is a complex and laborious method that is not suitable for measurement of CsA unbound concentration on a routine basis.

In the absence of active transport mechanisms, only file unbound portion of a drug is capable of diffusing across the membrane of salivary gland capillaries into saliva. Hence, the measured drug concentration in saliva should represent the unbound drug concentration. Cyclosporine is a neutral lipophilic molecule that diffuses into saliva via passive diffusion. Consequently, the total concentration of CsA in saliva correlates well with the plasma or blood unbound concentration, making CsA an ideal candidate for salivary therapeutic drug monitoring (STDM).

### MATERIALS AND METHODS

### Saliva and Blood Samples

For calibration purposes unstimulated drug-free saliva and EDTA whole blood samples were obtained from 10 and 2 healthy volunteers, respectively under the Institutional Review Board (IRB) Approval No. HU0203- 120 issued by the University of Rhode Island IRB. In addition, subsequent to signing of an informed consent form, 'blood and saliva samples were obtained from 15 kidney transplant recipients attending the outpatient clinic at the Rhode Island Hospital, Providence, Rhode Island. The unstimulated saliva was collected by asking patients to accumulate their saliva over 4-5 minutes and spit it into silanized plastic cups. To obtain a wide range of CsA concentrations in patient samples, no restriction on the time post CsA dose was implemented.

### Chemicals

Samples of CsA, cyclosporine C (CsC), and cyclosporine D (CsD) were donated by Novartis Parma AG (Basel, Switzerland). Ammonium acetate was obtained from Acros (Morris Plains, N J), a nd HPLC grade acetonitrile, heptane, isopropanol, and methanol w ere purchased from Fisher Scientific (Fair Lawn, NJ).

### Calibration Curves

A series of saliva-based calibrators (1, 10, 50, 100, 150, and 300 µg/L) and quality control standards (2, 75, and 200 µg/L) were prepared. All calibrators and quality control solutions were maintained at -20°C before use. A working internal standard/precipitating solution of 0.02 µg/L CsC in 94:6 vol/vol acetonitrile:water was prepared from the stock solution of CsC (1 µg/L) in methanol. For the measurement of CsA in whole blood, calibrators containing 5, 25, 50, 100, 250, 500, 1000, and 2500 µg/L of CsA and in-house quality control samples containing 60, 200, and 600 µg/L of CsA were prepared using blood samples from healthy donors.

### Sample Extraction

Before use, all glassware and plasticware was treated with AquaSil Siliconizing Fluid (Pierce, Rockford, IL). Saliva calibrators and patient samples were kept frozen at -20°C and thawed in a shaking water bath at 37°C. Patient or calibrator saliva samples (0.5 mL) were added to 1 mL of precipitant containing CsC in 1.5 mL polypropylene tubes. It is important that saliva be added to the precipitant because recovery of CsA was greatly affected if the order of addition was reversed. After 5 minutes of standing, the samples were vortex mixed for 1 minute and centrifuged for 7 minutes at 15,000 g, and the supernatants were extracted using solid- phase extraction.

The C₁₈ solid-phase extraction cartridges (200 mg, Waters, Milford, MA) were assembled on a manifold (Supelco Visiprep 24 DL) (Bellefonte, PA). The cartridges were pre conditioned with methanol and water (5 mL each). The supernatants were then added to the cartridges and left to drain under atmospheric pressure. Five minutes after all the supernatant had passed, the cartridges were washed sequentially with 5 mL de-ionized water, 2 mL of 50:50 vol/vol of methanol: water, and 2 mL heptane under atmospheric pressure. On passage of the heptane, the cartridges were subjected to vacuum at 20 psi for 15 minutes followed by the elution of the analytes using 3 mL of 50:50 vol/vol solution of isopropanol:heptane. Five minutes later, any remaining eluting solution left in cartridges was extracted using vacuum for 1 minute and pooled with the main eluent. The eluents were then dried at 60°C in a centrifugal evaporator (Thermosavant, Holbrook, NY), reconstituted with 300 µL methanol, vortex mixed for 5 minutes, and a 50-µL aliquot of this was injected onto the HPLC column.

The blood assay was based on a modified version of a method reported by Streit F, Armstrong VW, Oellerich M. Rapid liquid chromatography-tandem mass spectrometry routine method for simultaneous determination of sirolimus, everolimus, tacrolimus, and cyclosporin A in whole blood. Clin Chem. 2002;48:955-958.. Whole blood calibrators, quality controls, or patient samples (0.1 ml.) were vortex mixed for 30 seconds with 0.1 mL of a mixture of methanol and 0.3 mol/L ZnSO₄ (70:30 vol/vol) containing the internal standard CsC (250 µg/L) in 1.5-mL polypropylene tubes. The samples were centrifuged for 5 minutes at 15,000 g, and the supernatants were decanted. The samples were then recentrifuged for 1 minute at 15,000 g, and 150 µL was injected onto the column.

### HPLC Apparatus and Conditions

For saliva and blood assays, chromatography was performed on a Perkin Elmer series 200 liquid chromatography system consisting of an autosampler and 2 micropumps (Norwalk, CT). The analytic column consisted of an Aqua Perfect (150 x 3.0 mm; 5 µm) C₁₈ reversed-phase column (MZ Analysentechnik, Germany) maintained at 65°C using a column heater (FC-Flatron, Milwaukee, WI). A precolumn filter (Supelco, 1/16-inch, Peek 2 pm Frit, Bellefonte, PA) was used throughout the analyses.

For the saliva and blood assays, the mobile phase consisted of a 97:3 vol/vol mixture of methanol and 30 mmol/L ammonium acetate maintained at a flow rate of 0.5 mL/min. The run time was 5 minutes, and an equilibration, step of 1.5 minutes was included. For the whole-blood assay, the column was washed for 1 minute at a flow rate of 0.6 mL/min with an 80:20 vol/vol mixture of methanol and 30 mmoL/L ammonium acetate solution, followed by a 4-minute elution step at 0.6 mL/min with a 97:3 vol/vol mixture of methanol:30 mmoL/fL ammonium acetate. The mobile phase was then converted for 0.5 minutes to 80:20 vol/vol mixture of methanol: 30 mmoL ammonium acetate in preparation for the next injection.

### Mass Spectrometer Conditions

The API 2000 Triple Quadrupole Mass Spectrometer (LC-MS/MS system) (Sciex, Toronto, Canada) equipped with a turbo-ion spray source was used for all analyses. High-purity nitrogen gas obtained from a 240-L Liquid Nitrogen Dewar (MedTech Gases, Medford, MA) was used as nebulizer (Gas 1), auxiliary (Gas 2), and collision gases. For multiple reactant monitoring (MRM), the ammonium adducts [M+ NH₄]⁺ of CsA and CsC in the first quadrupole (Q₁) and their deaminated protonated adductions [M + H] ⁺ in the third quadrupole (Q₃) w ere s elected. System control and data acquisition w ere performed u sing the Analyst Service Pack version 1.2 software.

Source parameters including curtain gas pressure, temperature, ion spray voltage, and gas 1 and 2 pressures were optimized by flow injection analysis. Compound parameters including declustering potential, focusing potential, entrance potential, collision cell entrance potential, collision energy, and collision cell exit potential were optimized by infusing a 1 mg/L solution of CsA and CsC in mobile phase at a flow rate of 0.01 mL/min into the mass spectrometer. The flow injection analysis yielded the optimized source parameters of curtain gas: 10 psi, ion spray voltage 4500 V, temperature 350°C, gas 1 20 psi, and gas 240 psi. Ramping in the MPM mode further improved the sensitivity for both CsA and CsC ion pairs. A low declustering potential (<10 V) favored the entry of the ammonium fragment [M+ NH₄]⁺ for both CsA and CsC into the first quadrupole (Q,). Accordingly, the ramped parameters in MRM mode included a declustering potential (DP) 6 V, focusing potential (FP) 392 V, entrance potential (EP) 10 V, collision energy 40 V, and a collision cell exit potential 30 V for CsA and CsC.

### Method Validation

Assay validation for determination of CsA in saliva was performed according to the FDA guidelines for Bioanalytical Methods Validation for Human Studies. The lower limit of quantification (LLOQ) was defined at a signal-to-noise ratio of 10:1 and limit of detection (LOD) at signal-to-noise ratio of 5:1. The calibration curve was constructed by injecting triplicates of the extracted saliva-based calibrators inclusive of the LLOQ and the quality controls on 6 different study days. The CsA concentrations on the calibration curve ranged from 1 to 300 µg/L, and the quality controls were 2, 75, and 200 µg/L. A 1/x² weighted regression was used to fit the calibration data. The accuracy and interday coefficient of variation (%CV) of the method over the analytic range were determined from the back-calculated results obtained using the regression equation for each calibration curve on each of the 6 days.

The intraday %CV of the method was determined by measuring the CsA concentration in the quality control samples in 3 batches. This was repeated on 2 consecutive days. The interday %CV and inaccuracy were based on quality control concentration results obtained on at least 6 different days. The preset level of inaccuracy and imprecision of the assay method was set to be ≤ 15% for calibrators and QCs, except for the LLOQ, for which it could be ≤20%. The recovery of the analytes from saliva or blood matrix was determined by comparing the peak areas of the extracted samples, spiked with CsA and CsC against nonextracted CsA and CsC solutions.

Testing for freeze/thaw stability (3 cycles of freezing at - 20°C and thawing at room temperature [25° C] were performed on 3 aliquots each of 3 quality control samples) and short-term room temperature stability (3 aliquots of each of the quality control samples maintained at room temperature for 4 hours before sample extraction) of CsA in saliva were conducted according to the FDA guidelines.

The method for determination of cyclosporine in saliva using LC/MS/MS may be used to obtain better results by using additional extraction steps to increase the extraction efficiency and improve variability. These steps include three freeze-thaw cycles and sonicating the saliva samples to break-up the mucin structure.

### RESULTS

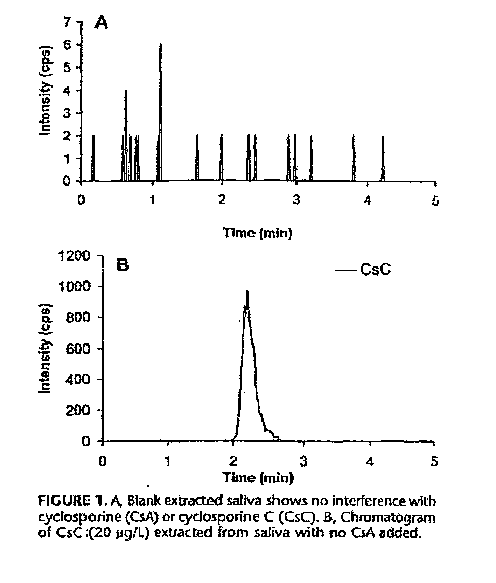

The mass transitions selected in the MRM were m/z 1219.9 →*m*/*z* 1202.9 for CsA and *m*/*z* 1235.94 →*m*/*z* 1218.9 for CsC. The retention times for CsA and CsC were approximately 2.4 and 2.2 minutes, respectively. The specificity of the assay indicated no interfering peaks at the retention time of CsA in blank saliva see Fig. 1A or blank saliva spiked with internal standard see Fig. 1B. The limit of detection was 0.6 µg/L, and the LLOQ was 1.0 µg/L with a signal-to-noise ratio of 10:1.

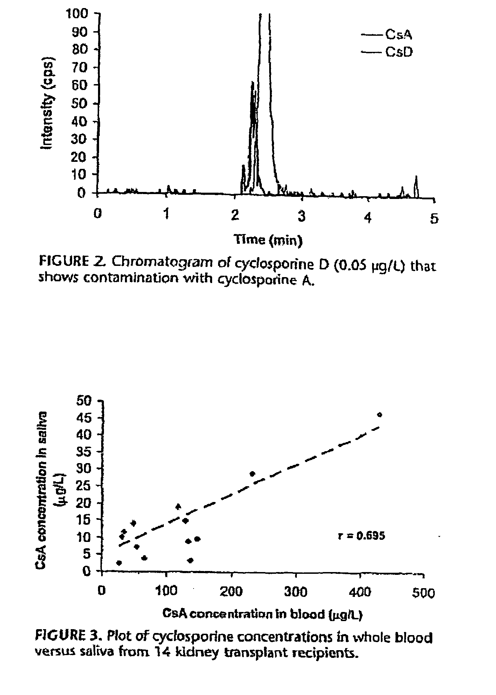

During the initial stage of method development, CsD was used as the internal standard for cyclosporine. It was observed that the samples of CsD were contaminated with CsA at a level of 0.2 µ/L as shown in Fig. 2. The contamination was reported to Novartis, and subsequently CsC was used as the internal standard. No trace of CsA was found in CsC samples.

The calibration curve was linear over the working concentration range of 1-300 µ/L with correlation coefficient values ranging from 0.9732 to 0.9968. The mean extraction recovery over the concentration range 1-300 µ/L was approximately 84.7 ± 2.6% for CsA (Table 1) and approximately 93.7 ± 4.4% for CsC. The accuracy and intra- and interday coefficients of variation (%CVs) are expressed in Table 1. The accuracy for the low (2 µ/L), medium (75 µ/L), and high (200 µ/L) quality controls (QCs) ranged from 92.0% to 104.7%. The intra- and interday %CVs for the QCs ranged from6.9% to 12.2% and 8.3% to 12.1%, respectively. Both intra- and interday %CVs were within 15%, the acceptable limit according to FDA guidelines. The accuracy and CVs of freeze/thaw and short-term stabilities are shown in Table 2. No systematic loss of sensitivity was observed in the absolute peak area measures as a result of sample storage.

| **TABLE 1.** Recovery, Accuracy, and Imprecision of the LC-MS/MS Assay | | | | |
|---|---|---|---|---|
| | | | **CV%** | |
| **Quality Control Concentration (µg/L)** | **Extraction Recovery (%) (n = 6)** | **Accuracy (%) (n = 6)** | **Intraday (n = 3)*** | **Interday (n** = **6)** |
| 2 | 86.7 ± 5.7 | 104.7 ± 8.2 | 12.2 | 8.3 |
| 75 | 85.7 ± 9.3 | 143.5 ± 7.0 | 11.3 | 8.5 |
| 200 | 81.7 ± 8.0 | 92.0 ± 11.1 | 6.9 | 12.1 |
| CV, coefficient of variation, | | | | |
| *Intraday CV was calculated using 3 batches of quality control concentrations on 2 consecutive days. | | | | |

| **TABLE 2.** Results of Stability Studies | | | | |
|---|---|---|---|---|
| | **Stability Criteria** | | | |
| **Quality Control Concentration (µg/L.)** | **Freeze/Thaw*** | | **Short-Term Stability†** | |
| | **CV%** | **Accuracy (%)** | **CV%** | **Accuracy (%)** |
| 2 | 7.9 | 96.2 | 2.6 | 92.2 |
| 75 | 10.7 | 101.2 | 8.1 | 98.5 |
| 200 | 7.6 | 104,7 | 2.2 | 99.8 |
| CV, coefficient of variation. | | | | |
| *Three freeze/thaw cycles were performed. | | | | |
| †The duration of time before extraction was 4 hours. | | | | |

Paired saliva and blood samples from 15 kidney transplant recipients were analyzed using the validated LC-MS/MS method. The results measured saliva and blood concentrations are depicted in Figure 3. No CsA concentration was detectable in either saliva or blood samples of 1 of the 15 patients. A reasonable agreement (correlation coefficient 0.695; *P*=0.006) was observed between the saliva and blood concentrations in the remaining 14 patients.

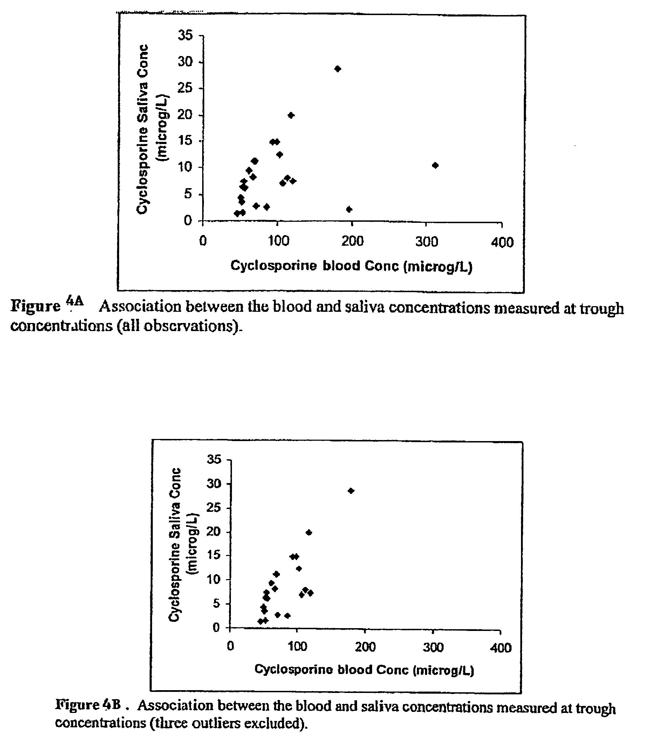

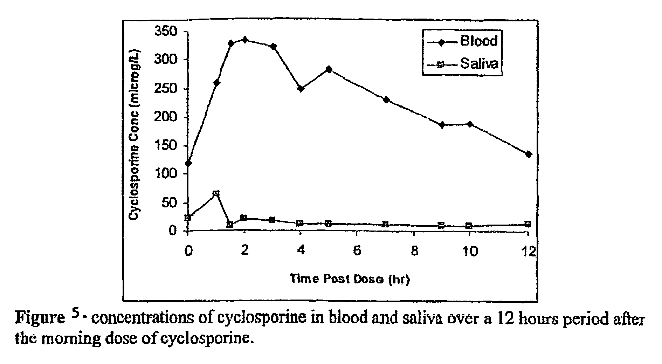

A pharmacokinetic study for cyclosporine in saliva and blood in 12 kidney transplant recipients was conducted. In this study all patients took a dose of cyclosporine in the morning and the concentrations of cyclosporine in blood and saliva were measured for 12 hours after the dose. Figures 4A and 4B depict the association between trough concentrations of cyclosporine (obtained before the dose and at 12 hours after the dose) in blood and saliva (24 observations in total). With the exception of three blood-saliva pairs the remaining concentrations correlates very well (correlation coefficient = 0.529, n=24, P value 0.008 versus correlation coefficient=0.77 n P value <0.0001). Also the Figure 5 represents the concentrations of cyclosporine in blood and saliva over the 12 hours period after the dose.

An efficient method for extracting CsA from saliva was discerned. Because of the viscous and gluey nature of the saliva matrix, the recovery of CsA extraction was around 20% using liquid-liquid or solid-phase extraction methods previously reported for blood assays. Addition of the matrix to the protein precipitant solution significantly improved the recovery of CsA extraction. Silanizing all glassware and plasticware increased the extraction efficiency.

Almost exclusively previous mass spectrometry methods for CsA used CsD as internal standard (Streit F, Armstrong VW, Oellerich M. Rapid liquid chromatography-tandem mass spectrometry routine method for simultaneous determination of sirolimus, everolimus, tacrolimus, and cyclosporin A in whole blood. Clin Chem. 2002; 48:955-958). Keevil and colleagues also used the commercially available molecule, ascomycin as the internal standard for CsA. Originally we had used CsD, during which we noticed that CsD was contaminated with CsA (Fig. 2). The CsA area under the peak ac counted for almost 10% of the CsD area under the peak for 0.05 µg/L of CsD. This contamination has not been reported previously in the literature. Novartis Pharmaceuticals was in formed about the contamination, and they kindly donated a sample of CsC that showed no contamination with CsA. Although this contamination may not be problematic for HPLC analysis, it can potentially generate erroneous measurements near the assay LLOQ for the LC-MS/MS. The attempts to use ascomycin as the internal standard did not produce adequate recovery because of the differences between the molecular structures of ascomycin and CsA. It is recommended that CsC be used instead of CsD to avoid introducing bias in calculation of unknown CsA, especially around the LLOQ concentrations.

Thus there is the development and validation of a sensitive and specific LC-MS/MS for determination of CsA in saliva.

CsA concentrations in blood and saliva from 15 kidney transplant recipients were measured. The purpose of these measurements was to test the LC-MS/MS method and to obtain a crude approximation of the correlation between CsA concentrations in blood and saliva. Because the original intention was to have a wide range of concentration, no stipulation on time of sampling was implemented as long as the blood and saliva samples were obtained within a 10-minute time period. The saliva and blood concentrations broadly correlate with each o ther. Also, the test conducted herein included no documentation on the existence of gingival hyperplasia in the patients. This condition, which is one of the most important side effects of CsA therapy, is associated with an increased plaque accumulation and bleeding in mouth, which may lead to erroneous measurement of CsA concentration in saliva. Because the existence of traces of w hole blood in saliva can over estimate the salivary concentration of CsA, in any future study of CsA saliva determination it is imperative to document the traces of blood in saliva.

Non stimulated saliva was collected from kidney transplant recipients. The stimulation of salivary flow by placing citric acid on the tongue or chewing paraffin wax may generate more saliva in a shorter period of time. However, the potential problems of changes in the salivary pH and adsorption to the paraffin wax must be evaluated for any specific drug before this technique can be employed in clinical studies. In addition, it is theoretically possible that plasma and saliva equilibration may not be achieved when a larger volume of saliva is produced in a short period of time; therefore, the saliva concentration may not represent the true saliva-to- plasma concentration ratio.

Thus there is the development and validation of a sensitive and specific IC-MSIMS m for determination of CsA in saliva.

A sensitive, specific, precise, and accurate method for the measurement of CsA in saliva is reported.

## Claims

1. A method of monitoring cyclosporine in saliva, said method comprising:
freezing and thawing an obtained saliva sample;
sonicating said saliva sample;
adding said saliva sample to a solvent and water mixture to determine the concentration of cyclosporine in said saliva.

2. The method of claim 1 wherein the solvent is acetonitrile.

3. The method of claim 1, wherein monitoring of cyclosporine in saliva is a substitute for the monitoring of cyclosporine in blood.

4. The method of claim 1, wherein the measurement of cyclosporine concentration in human saliva is sensitive with a lower limit of quantification of 1 ng/mL allowing a measurement of very low concentrations.

5. The method of claim 1 wherein the method is specific for parent cyclosporine molecule such that the measured concentrations represent the concentration of parent drug but not inactive metabolites.

6. The method of claim 1, wherein saliva pre-treatment with sonication improved recovery and consistency of cyclosporine extraction from saliva.

## Patentansprüche

1. Verfahren zum Überwachen von Cyclosporin in Speichel, wobei das Verfahren umfaßt:
Einfrieren und Auftauen einer erhaltenen Speichelprobe;
Beschallen der Speichelprobe;
Hinzufügen der Speichelprobe zu einer Lösungsmittel-Wasser-Mischung, um die Konzentration von Cyclosporin in dem Speichel zu bestimmen.

2. Verfahren nach Anspruch 1, worin das Lösungsmittel Acetonitril ist.

3. Verfahren nach Anspruch 1, worin das Überwachen von Cyclosporin in Speichel ein Ersatz für das Überwachen von Cyclosporin in Blut ist.

4. Verfahren nach Anspruch 1, worin die Messung einer Cyclosporin-Konzentration in menschlichem Speichel eine Empfindlichkeit mit einer unteren Grenze der Quantifizierung von 1 ng/mL aufweist, was eine Messung von sehr niedrigen Konzentrationen erlaubt.

5. Verfahren nach Anspruch 1, worin das Verfahren spezifisch für Ausgangs-Cyclosporin-Molekül ist, so daß die gemessenen Konzentrationen die Konzentration von Ausgangswirkstoff, aber nicht von inaktiven Metaboliten darstellen.

6. Verfahren nach Anspruch 1, worin die Vorbehandlung des Speichels mit Beschallung die Ausbeute und Konstanz von Cyclosporin-Extraktion aus Speichel verbessert.

## Revendications

1. Procédé de contrôle de la cyclosporine dans la salive, ledit procédé comprenant :
la congélation et la décongélation d'un échantillon de salive obtenu ;
la sonication dudit échantillon de salive ;
l'addition dudit échantillon de salive à un mélange de solvant et d'eau pour déterminer la concentration de cyclosporine dans ladite salive.

2. Procédé selon la revendication 1, dans lequel le solvant est l'acétonitrile.

3. Procédé selon la revendication 1, dans lequel le contrôle de la cyclosporine dans la salive est un substitut au contrôle de la cyclosporine dans le sang.

4. Procédé selon la revendication 1, dans lequel la mesure de la concentration de la cyclosporine dans la salive humaine est sensible avec une limite inférieure de quantification de 1 ng/ml permettant une mesure de concentrations très basses.

5. Procédé selon la revendication 1, où le procédé est spécifique de la molécule parente de cyclosporine de telle manière que les concentrations mesurées représentent la concentration du médicament parent mais pas des métabolites inactifs.

6. Procédé selon la revendication 1, dans lequel le prétraitement de la salive par sonication a amélioré la récupération et la régularité de l'extraction de cyclosporine à partir de la salive.
